# EUROPEAN PATENT APPLICATION

(11) **EP 3 460 067 A1**
(43) Date of publication of application: **27.03.2019**
(21) Application number: 17192457.4
(22) Date of filing: 21.09.2017
(51) Int. Cl.: C12P 7/40, C12P 7/42, C12P 7/44, C12P 7/58, C12P 19/02, C12P 7/24, C12N 9/04

(54) **PROCESS FOR ENZYMATIC OXIDATION OF LINEAR CARBOHYDRATES**

(30) Priority: 20.09.2017 EP 17192258
(71) Applicant: Technische Universität München, 80333 München (DE)
(72) Inventor: SIEBER, Volker, 85405 Nandlstadt (DE); BEER, Barbara, 94315 Straubing (DE); PICK, André, 94315 Straubing (DE)
(74) Representative: Leißler-Gerstl, Gabriele

(57) **Abstract**

A process for enzymatic oxidation of a terminal group of a linear carbohydrate is disclosed, wherein a carbohydrate of formula (I): **TU-X-Y-TOG,** wherein TU is a terminal unit selected from -CH₂OH, -CHO, -COOH, and -C₁-C₄ alkyl; X is a linear carbohydrate chain comprising 0 to 18 units [CR¹R²], wherein in each unit R¹ and R² independently can be hydrogen, hydroxyl, C₁-C₄ alkyl, or can together form a keto group, Y is -CHOH, -CHNH₂ or -CHSH and TOG is an oxidzable terminal unit selected from -CH₂OH and -CHO; is contacted with an NAD⁺ dependent short chain dehydrogenase/reductase for oxidation of TOG.

## Description

### FIELD OF THE INVENTION

The present invention relates to a novel process for enzymatic oxidation of linear carbohydrates and to producing rare sugars and sugar acids obtained by using an enzyme isolated from *Sphingomonas species A1.*

Sugars and sugar derivatives play an important role in biochemical research, pharmaceutical drug discovery and food industry as they are involved in a myriad of metabolic processes, among these are signaling, cellular recognition and processes that are central to human health. Unfortunately, only a small number of all possible monosaccharides (D-glucose, D-galactose, D-mannose, D-fructose, D-xylose, D-ribose, and L-arabinose according to the International Society of Rare Sugars (ISRS)(Granström et al., 2004)) are found in nature in sufficient amounts to allow their commercial exploitation. Consequently, so-called rare sugars have to be produced by (bio)chemical processes starting from cheap and widely available substrates. It is known to use enzymes for producing rare sugars, such as enzymes of the classes aldolases, keto-aldol isomerases, epimerases, and oxidoreductases (Izumori, 2006; Li *et al.,* 2015). The class of oxidoreductases comprises dehydrogenases/reductases (EC 1.1.1.-) that catalyze oxidations and reductions of carbohydrates. They can be classified into short-chain (SDR), medium-chain (MDR), and long-chain (LDH) dehydrogenases/reductases according to the length of the amino acid chain. SDRs consist of about 250 to about 300 amino acids and contain two domains: a cofactor-binding domain and a substrate-binding domain. While the cofactor-binding domain is usually conserved, the substrate-binding domain varies and determines the substrate specificity (Kavanagh et al., 2008). These enzymes use NADP(H) or NAD(H) as a cofactor. Enzymes of the SDR family are involved in carbohydrate metabolism (Koropatkin and Holden, 2005; Zhang et al., 2009), and other important mechanisms, such as signal transduction (Benach et al., 2002; Svegelj et al., 2012), and fatty-acid synthesis, where (keto-acyl-(acyl carrier proteins)) and (enoyl-acyl-(acyl carrier proteins)) are substrates (Kim et al., 2011; Lee et al., 2007; Price et al., 2004; Zaccai et al., 2008). More than 120,000 enzymes in the SDR family are registered in UniProtKB (January 2017); they are found in organisms ranging from bacteria to humans. New enzyme members of this superfamily are identified every year (almost 42,000 new entries in UniProt in 2016), enzymes that are active in metabolising a vast number of substances.

SDR enzymes are important because of their physiological role, they can have a narrow to broad substrate specificity. (Hirano et al., 2005; Pennacchio et al., 2010; Stekhanova et al., 2010). Many SDR enzymes show a so called substrate promiscuity, i.e. have a broad scope of substrate use, which is widely accepted for being the starting point for enzyme divergent evolution (Khersonsky and Tawfik, 2010; O'Brien and Herschlag, 1999). New functions are developed by these non-native, secondary activities; as has been shown in experimental laboratory work using directed evolution (Kazlauskas, 2005; Turner, 2009).

To identify these secondary activities, enzymes can be isolated from cells and can be exposed to reaction conditions or substrates that do not occur *in vivo.* When used under such non-natural conditions or with unusual substrates, respectively, unforeseeable reactions can occure, they might for example catalyze reactions or show specificities that were not expected.

Enzymes are important in industrial biotechnology for the environmentally friendly synthesis of fine as well as bulk chemicals (Arora et al., 2014) and it is a demanding task to find enzymes that catalyze useful reactions and thereby allow to obtain substances that are not available in reasonable amounts until now. Therefore, it is an object of the invention to find enzymes having new activities that are useful in the synthesis of non-native substances or for the conversion of non-native intermediates of an artificial metabolic pathway for *in vivo* but also *in vitro* applications. It is desirable to find enzymes catalyzing valuable reactions *in vitro,* thereby avoiding constraints such as cell-viability or transportation across cell-boundaries. Furthermore, it is desirable to identify enzymes that have specificity for a reaction, for a structural entity or a functionality, to allow introduction or reaction of a specific group without changing the rest of a molecule. On the other hand it is desirable that the activity of an enzyme is not confined to one specific molecule or specific conditions. Thus what is desired is flexibility regarding the substrate and reaction condition but not regarding the site. Moreover, it is desirable to avoid side reactions, which are a drawback of promiscuous enzymes.

An NADH/NADPH-dependent SDR involved in alginate metabolism for the reduction of 4-deoxy-L-erythro-5-hexoseulose (DEHU) to 2-keto-3-deoxy-D-gluconate (KDG) has been identified in *Pseudomonas* (Preiss and Ashwell, 1962) and *Vibrio splendidus* (Wargacki et al., 2012), and recently characterized in detail from *Sphingomonas species* A1 (Takase et al., 2014; Takase et al., 2010), *Flavobacterium species* strain UMI-01 (Inoue et al., 2015) and abalone (Mochizuki et al., 2015). The latter two studies also addressed the question of substrate scope. However, no activity was observed for the reduction of other substrates except DEHU. Moreover, it is indicated in the prior art that the oxidation of KDG to DEHU is unfavorable, which might be due to the physiological task of SpsADH. DEHU is toxic to cells and therefore needs to be metabolized to non-hazardous KDG by the cells (Hashimoto et al., 2010).

It was an object of the present invention to provide means for targeted oxidation of carbohydrates to produce valuable products that are not found in nature and can be used in pharmaceutical, cosmetic and food applications. Moreover, it was the object of the present invention to provide means to produce sugar and sugar derivatives with rare occurring conformation, and it was an object of the present invention to provide a method for producing L-sugars and sugar acids.

It was surprisingly found that an enzyme that has been isolated from *Sphingomonas species A1* (SpsADH, ID: SPH3227, PDB: 4TKM) or a homolog thereof can be used for oxidation reaction on native as well as non-native compounds. Although this enzyme was already known, it was only used and known for reduction reactions of DEHU. However, surprisingly this enzyme is reactive as catalyst for the oxidation of compounds that have a structure as defined in formula I. In addition, it was found that this enzyme is specific for the oxidation of terminal hydroxyl and aldehyde groups, whenever they are adjacent to a C-atom with an -OH, SH or -NH2 group bound thereon. In particular, the inventors found, that the enzyme has specificity for terminal hydroxyl or aldehyde groups with an adjacent C-atom with a hydroxyl group bound thereon and in particular when this adjacent C-atom is a chiral C-atom, in a configuration as defined below. Therefore, this enzyme is highly valuable as it allows to obtain sugar and sugar derivatives that are not or only with high cost and effort obtainable.

Thus, the present invention is concerned with a process for enzymatic oxidation of a carbohydrate, wherein a carbohydrate of formula (I):

**TU-X-Y-TOG**

wherein TU is a terminal unit selected from CH₂OH, CHO, COOH, and C₁-C₄ alkyl;
X is a linear carbohydrate chain having 0 to 17 units [CR¹R²], wherein in each unit R¹ and R² independently can be hydrogen, hydroxyl, C₁-C₄-alkyl, carboxyl or wherein R¹ and R² together can form a keto group;
Y is -CHOH; -CHSH or -CHNH₂ and
TOG is a terminal oxidizable unit -CH₂OH or-CHO;
is contacted with an NAD⁺ dependent short chain dehydrogenase/reductase (SDR) or a homolog thereof for oxidation of TG. The enzyme preferably is SpsADH from *Sphingomonas species A1* or a homolog thereof.

It was surprisingly found that the SDR from *Sphingomonas species A1,* in the following also called SpsADH, specifically oxidizes a terminal hydroxyl or aldehyde when this group has an adjacent C-atom to which a thiol group, an amino group or a hydroxyl group is bound, in particular when this adjacent C-atom is a chiral C-Atom, in a configuration as defined below. Therefore, this enzyme can be used to produce new compounds and compounds that are rarely found in nature and which have very valuable properties.

The enzyme SpsADH consists of 258 amino acids, has 64 % sequence similarity to its NADP⁺-dependent isoenzyme and high sequence identity (40-71 %) to 3-ketoacyl-CoA-reductases. Surprisingly although SpsADH was proven promiscuous (a variety of substrates were recognized: most aldonic acids and uronic acids, polyols and some sugars), an interplay of specificity and promiscuity is given based on the carbon getting attacked. The carbon getting attacked is dependent on the presence and configuration of hydroxyl groups in the group adjacent to that carbon. It was shown that the presence of a hydroxyl, thiol, or amino group in particular a hydroxyl group in the adjacent group is mandatory. Terminal groups adjacent to a -CH2 group were not oxidized. Furthermore for oxidative efficiency a certain stereochemical configuration was favorable. The inventors found for example that when contacting the enzyme of the present invention with D-sorbitol, only L-gulose is obtained, but not a mixture of L-gulose and D-glucose.

Rare sugars and sugar derivatives that can be obtained from abundant sugars are of great interest to the pharmaceutical industry and others. The invention describes carbohydrates useful as substrates for the oxidation through the known dehydrogenase/reductase of *Sphingomonas species* A1 e.g. aldonic acids, uronic acids aldoses and polyols. The resulting products are aldaric acids, aldonates and rare sugars, respectively, which are top value-added chemicals derived from renewable resources or play an important role in biochemical and pharmaceutical research. As an example, the applicability of this enzyme for the synthesis of L-gulose from D-sorbitol in an in vitro system using an NADH oxidase for cofactor recycling is shown.

### BRIEF DESCRIPTION OF THE DRAWINGS

Figure 1 shows the native reaction of SpsADH and the reaction with analog substrates. Top: SpsADH is involved in alginate metabolism for the reduction of 4-deoxy-L-erythro-5-hexoseulose (DEHU) to 2-keto-3-deoxy-D-gluconate (KDG). Bottom: Activity of SpsADH with substrate analogs of KDG: D-gluconate, 2-deoxy-D-gluconate, and 3-deoxy-D-gluconate is exemplified.
Figure 2 shows the activity of SpsADH with D-aldonates. Carbon chain lengths of three to six were tested (gray background) at the indicated substrate concentrations. The kinetic parameters vₘₐₓ (solid black box) and K_{M} (dashed box) were determined for C5- and C6-aldonates of the erythro group.
Figure 3 shows the activity profile for polyols. Oxidation of polyols can theoretically take place at either C1 or C6. For D-sorbitol, specific oxidation of C6 was proven, resulting in the formation of L-gulose.
Figure 4 shows the synthesis of L-gulose using SpsADH. Starting from D-sorbitol, 300 mM L-gulose was produced by SpsADH within 334 h. This gives a space-time yield of 0.9 mM h⁻¹.
Figure 5 shows the oxidation of D-sorbitol to L-gulose Top: L-Gulose control; Middle: Control reaction without SpsADH (only UV-Systempeaks); Bottom: Reaction of SpsADH with D-sorbitol: only L-Gulose (0,105 g/l, 5,8 mM), no D-glucose was detected

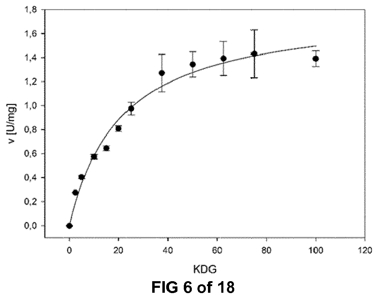

Figure 6 shows the kinetics of SpsADH for KDG-oxidation.
Figure 7 shows the detection of uronic acids by HPLC/MS

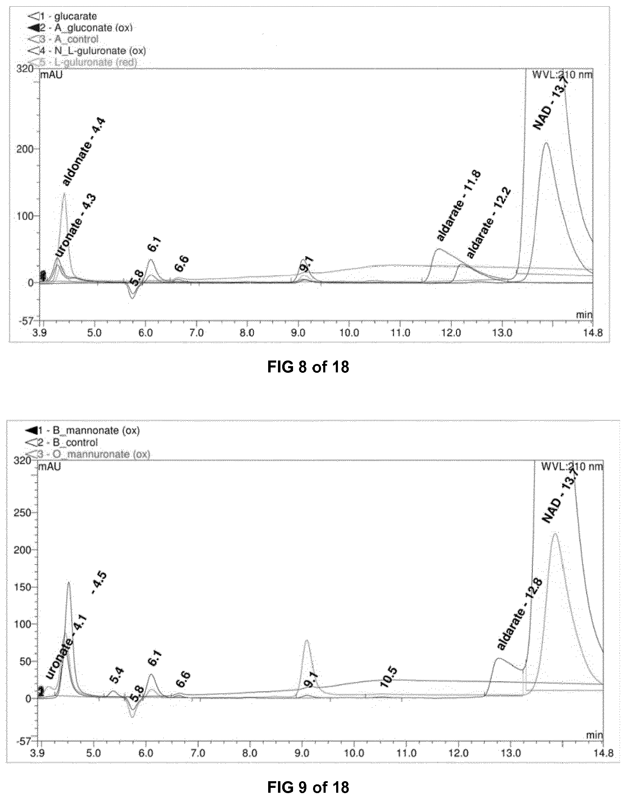

Figure 8 shows the oxidation of D-gluconate to L-guluronate and glucarate
Figure 9 shows the oxidation of D-mannonate to D-mannuronate and mannarate
Figure 10 shows the oxidation of D-allonate to L-alluronat and allarate
Figure 11 shows the oxidation of D-altronate to D-taluronate and altrarate
Figure 12 shows the oxidation of D-galactonate and L-galacturonate to galactarate
Figure 13 shows the oxidation of D-talonate to L-altruronate and talarate

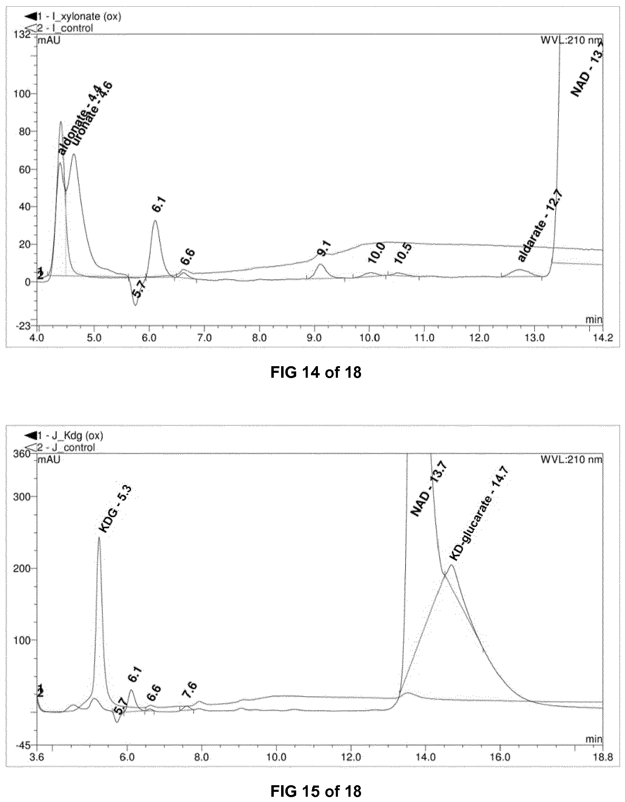

Figure 14 shows the oxidation of D-xylonate to L-xyuronate and xylarate
Figure 15 shows the oxidation of Keto-deoxy-D-gluconate (KDG) to keto-deoxy-glucarate
Figure 16 shows the oxidation of uronic acids
Figure 17 shows the purification of L-gulose by cation exchange chromatography
Figure 18 shows the codon optimized DNA sequence. Start- and Stopp-codons in bold letters, restriction sites in underlined letters.

### DEFINITIONS

The present invention is concerned with the oxidation of carbohydrate compounds. The term "carbohydrate" refers to compounds that comprise carbon atoms and hydrogen atoms, but also oxygen atoms and can also comprise other atoms like nitrogen in minor amounts. A typical carbohydrate of the present invention is a sugar, sugar acid or other sugar derivative.

"Terminal oxidizable unit" or "TOG" refers to one of the terminal carbon groups of a compound of formula (I) which is to be oxidized by the enzyme of the present invention and which is adjacent to Y.

"Terminal unit" or "TU" refers to the other terminal carbon group of formula (I) that is bound to the carbohydrate chain X or is bound directly to Y. The terminal unit TU is not to be oxidized by the enzyme of the present invention.

Carbohydrates as used in the present invention, comprise chiral groups. The nomenclature for defining the stereochemical configuration at the carbon atom of Y adjacent to the terminal oxidizable carbon group TOG can be identified by the Fischer projection. For the case that Fischer projection is applied and the terminal carbon group TOG is the lowermost carbon in this projection the stereochemistry of the molecule is referred to as "D-configuration" (D-CHOH). "D-CHOH", therefore, refers to a hydroxyl carbon group in the chain which has D-configuration as defined here. For the case that the terminal carbon group TOG is the topmost carbon in this projection the preferred stereochemistry of the molecules of the invention is the L-configuration (L-CHOH). Furthermore as used herein "R configuration" or "stereochemical configuration R" or "R-CHOH" of the C-atom adjacent to the terminal carbon group TOG means the stereochemistry wherein the priority of the terminal group TOG within the R/S nomenclature is higher than that of group -X-TU. The "S-configuration" or "S-CHOH" in the sense of the invention, is given when the priority of the terminal group TOG is lower than that of group -X-TU.

In the definition of the present invention the term "adjacent" refers to the carbon atom directly bound to the terminal carbon group TOG.

A term "unit" [CR¹R²] defines one unit of a carbohydrate chain X, wherein R¹ and R² are as defined in claim 1. Examples for a unit are a -CHOH unit as in a sugar molecule, a - C=O or keto group, as in a sugar molecule, an unsubstituted CH₂ group or another substituted carbon group.

"Chiral center" refers to a carbon atom which has four different substituents.

"NAD-dependent" refers to an enzyme, that uses NAD⁺ as co-factor.

The term "NAD⁺-dependent short chain dehydrogenase / reductase enzyme" as used in the present descriptionrefers to an enzyme that has the specificity of an NAD⁺-dependent short chain dehydrogenase/reductase isolated from *Sphingomonas species A1,* also referred to as SpsADH, which is also found in *Vibrio, Pseudomonas, Flavobacterium* or *Abalone.* Moreover it also comprises homologs of this enzyme.

A homolog of SpsADH is an NAD⁺-dependent short chain dehydrogenase/reductase having at least 80% of the activity of SpsADH. The activity of SpsADH is oxidizing a terminal group TOG adjacent to a -CHOH, -CHSH, or -CHNH₂ group. A homolog can be an enzyme isolated from another organism having a similar specificity or an enzyme that has been modulated to optimize activity and/or specificity.

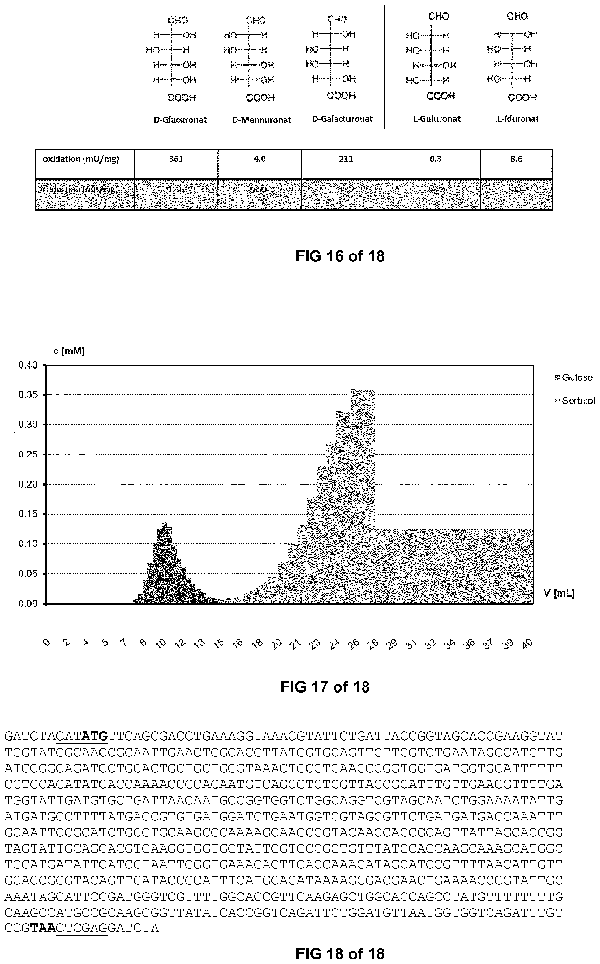

The term "NAD⁺-dependent short chain dehydrogenase/reductase from *Sphingomonas species A1" or "SpsADH"* refers to an enzyme with the amino acid sequence of BAP40335.1 (GenBank ID; Figure 18), or a homolog thereof having a similar activity or an SpsADH enzyme that has been modulated to optimize activity and/or specificity.

The term "STY [g L⁻¹ d⁻¹]" means space-time yield and is the amount of product formed [g] per liter [L] within one day [d].

In case of C6-aldonic acids implications for specificity can be exemplified: The activity with D-gluconate is more than 1000 times higher compared to the corresponding polyol sorbitol (1.3 U/mg and 1.7 mU/mg, respectively). Aldonic acids with shorter chain length, have lower activity.

The resulting uronic acids can be further oxidized by SpsADH. The SpsADH, being an uronate dehydrogenase with such a broad substrate scope, is surprising. The resulting aldaric acids are considered top-value added chemicals to be obtained from biomass (Werpy & Petersen, 2004). Here, glucuronate was used as substrate, but glucose could also be converted with glucose dehydrogenase and SpsADH. By abstracting the cofactor NADH, the reaction equilibrium can be shifted. Addition of NADH oxidase to the oxidation of gluconate resulted in formation of glucarate.

One embodiment of the invention is a process for producing an aldose from a polyol comprising an enzymatically catalyzed oxidizing step wherein the enzyme is SpsADH. It could be shown that the rare sugar L-gulose can be produced from D-sorbitol with higher yield as known from other single step conversion processes by using the enzyme of the present invention or a homolog thereof. A glucose-6-phosphate isomerase from *P. furiosus* is also described for the production of L-gulose from L-idose, however incomplete conversion as well as the side-product L-sorbose would make downstream processes more laborious (Yoon et al., 2009) than the approach using SpsADH.

In summary, inventors showed that SpsADH exhibits substrate promiscuity, when challenged with non-native concentrations. Even with the activities found here being very low (mU/mg range), the long half-life of SpsADH allows conversion of significant amounts up to full conversion.

The present invention relates to a process for enzymatic oxidation of a carbohydrate compound. The enzyme used for this reaction is an NAD⁺-dependent short chain dehydrogenase / reductase enzyme. Preferably the NAD⁺-dependent short chain dehydrogenase/reductase is from *Sphingomonas species A1* or a homolog thereof. This enzyme specifically oxidizes hydroxyl or aldehyde at the terminal carbon atom when the next or adjacent carbon atom carries a hydroxyl group. The hydroxyl or aldehyde group of a terminal carbon group is hardly or not oxidized when this group is bound to a CH₂ unit. The aldehyde group at the terminal carbon atom is oxidized especially when it is free and not in the form of a hemiacetal. Thus, this enzyme has high specificity for oxidizing a configuration with a hydroxyl or aldehyde group with an adjacent -CHOH, -CHSH, or -CHNH₂ moiety, in particular -CHOH. Furthermore, it was found that the enzyme favors those compounds where the C-atom in the adjacent group has a stereochemical configuration as defined above. No reducing activity or hardly any activity was found, when the carbohydrate was in hemiacetal form, for example with C₅ and C₆ sugar molecules in hemiacetal form.

Furthermore, it was found that only the terminal hydroxyl or aldehyde group is oxidized, but no hydroxyl or keto group in the chain. Therefore, the enzyme because of its specificity is very useful. For example, this enzyme allows producing L-gulose, which is rarely found in nature and is an interesting compound in the pharmaceutical field.

The process comprises that a carbohydrate of formula (I) is contacted with the enzyme of the present invention, i.e. an NAD⁺ dependent short chain dehydrogenase/reductase or a homolog thereof. The enzyme catalyzes oxidation of TOG.

The compound to be oxidized is a compound of formula (I): TU-X-Y-TOG. In this formula TOG is the terminal carbon group or unit to be oxidized, namely a hydroxyl group or an aldehyde group. The enzyme either oxidizes a hydroxyl group to an aldehyde group or an aldehyde group to a carboxyl group. The specificity of this reaction can be further influenced by using a compound modulating co-factor NAD⁺.

The unit TOG is -CH₂OH or -CHO, it should not be substituted by other groups, to not hinder the contact with the enzyme.

The second important part of the carbohydrate of the present invention is Y, a carbon group with OH, SH, or NH₂, which is directly adjacent to the terminal carbon group TOG to be oxidized. Y is preferably -CHOH, and preferably has a configuration as defined above for the invention. In experiments it was found that the favored configuration of the molecules to be oxidized allows to use a stereochemical mixture of substrates and nevertheless to obtain the favored configuration for the oxidized product.

The rest of the molecule, TU and X, is not as critical. TU is the terminal unit on the other side of the molecule which is not involved in the oxidation reaction and is selected from -CH₂OH, -CHO, -COOH, and -CH₃; X is a linear carbohydrate chain with 0 to 17 units, wherein the units can be the typical units of a sugar molecule, i.e. -CHOH, or other substituted or unsubstituted groups, such as CH₂ or carbon substituted by 1 or 2 hydroxyl groups, alkyl groups etc. At least one of the 0 to 17 units can be a keto group. In other words, the substrate either comprises TU, Y and TOG or additionally comprises X which is a chain of up to 17 units [CR¹R²], wherein in each unit R¹ and R² independently can be hydrogen, hydroxyl, C₁-C₄-alkyl, carboxyl or wherein R¹ and R² together can form a keto group. Preferably R¹ and R² each independently can be hydrogen or hydroxyl. The more remote a unit is from the terminal carbohydrate group to be oxidized, the less critical the structure is. The carbohydrate of the present invention, thus, at least has 3 units, i.e. TU-Y-TOG and can have up to 20 units, i.e. TU-[CR¹R²]₁₇-Y-TOG. Preferably, the carbohydrate of formula (I) has 3 to 8 units, in particular 3 to 6 units. Thus, in total the compound of the present invention preferably has a chain with 3 to 8 carbon atoms.

In a preferred embodiment, the compound to be oxidized is a sugar or sugar derivative. Some examples for molecules that have been successfully oxidized by using the SpsADH enzyme of the present invention or a homolog thereof are sugar alcohols like sorbitol, mannitol or xylitol and aldonic acids like gluconate, mannonate, allonate, altronate, arabonate, xylonate or talonate.

The enzyme of the present invention oxidizes the oxidizable group TOG of any substrate of formula (I) independent from the other terminal group TU. When a sugar alcohol is the substrate to be oxidized, i.e. a carbohydrate with -CH₂OH groups on both ends, only TOG, i.e. the -CH₂OH unit adjacent to Y is oxidized to yield an aldose. As the enzymes specifically oxidizes that terminal group that has a D-CHOH group adjacent, it is possible to obtain L-gulose from D-sorbitol.

When a aldonic acid is the substrate to be oxidized, wherein one terminal group is -CH₂OH and the other is -COOH, the terminal -CH₂OH group is oxidized to -CHO. The enzyme oxidizes preferably the TOG of moleciles with a D-CHOH adjacent group in high yield and therefore it is possible to obtain uronic acids i.e. L-guluronate, D-mannuronate, L-alluronate, D-taluronate, L-altruronate and L-xyluronate. The -CHOgroup can be oxidized further by the enzyme of the invention to mannarate, allarate, altrarate, galactarate, talarate and xylarate.

When a compound of formula (I) is used with a terminal -CHO group, i.e. an aldehyde group, the aldehyde is oxidized to yield a carboxy group. Thus, with the method of the present invention it is possible to specifically prepare aldaric acids. Examples for a substrate for this embodiment are L-guluronate, D-mannuronate, L-alluronate, D-taluronate, L-altruronate and L-xyluronate.

Of particular interest is the oxidation of uronic acids to yield aldaric acids which are deemed to be highly valuable but cannot be obtained in specific and easy manner with methods of the prior art. Such aldaric acids, i.e. bifunctional molecules can be used as units for polymerization and for preparing hyperbranched polyesters. Moreover, the metabolite α-ketoglutarate can be prepared by an in vitro enzymatic cascade (Beer et al., 2017). By depleting co-factor NADH the equilibrium of the reaction can be shifted. It has been found, that addition of NADH oxidase to the oxidation reaction of gluconate improves the yield of glucarate.

Moreover, as outlined above, the method of the present invention is an elegant way for producing L-gulose. By using the ubiquitous sugar alcohol sorbitol, and by oxidation of the terminal hydroxyl group, L-gulose can be obtained in easy manner and in a single step process. Approaches known from the prior art, where for example L-gulose has been prepared by using mannitol dehydrogenase from *Apium graviolens* in a recombinant *E. coli* strain resulted in a low yield of only 0.9 g L⁻¹d⁻¹ (Woodier et al., 2010). In contrast thereto, according to the present invention with the specific enzyme, a yield of 3.9 g L⁻¹d⁻¹ is obtained.

The enzyme used in the process of the present invention is an NAD⁺-dependent short chain dehydrogenase/reductase. In particular, an enzyme used in the process of the present invention is an enzyme that has the specificity of an NAD⁺-dependent short chain dehydrogenase/reductase isolated from *Sphingomonas species A1,* also referred to as SpsADH, which is also found in *Vibrio, Pseudomonas, Flavobacterium* or *Abalone* or a homolog thereof, in particular a homolog having at least 80% of the activity of SpsADH. The activity of SpsADH is oxidizing a terminal group TOG adjacent to a -CHOH, -CHSH, or CHNH₂ group. Preferably, an NAD⁺-dependent short chain dehydrogenase/reductase from *Sphingomonas species A1" or "SpsADH"* with an amino acid sequence of BAP40335.1 (GenBank ID; Figure 18) has been found useful. Furthermore, as is known to the skilled person, starting from the above sequence, the enzyme can be improved using techniques like genetic engineering. Thus, the present invention also comprises a method using a genetically engineered enzyme which has been obtained from SpsADH by refining or adapting it to the substrate.

The enzyme used according to the present invention is very stable and has a satisfying turnover rate. Stability of an enzyme is important to ensure complete conversion by extended incubation. When analyzing the enzyme of the present invention it was found that the enzyme at 25° has a half-life of about 20 hours. In the case of oxidation of D-gluconate to yield L-guluronic acid, a turnover rate of 28,300 was found.

The oxidation of the carbohydrates of formula (I) is carried out by contacting the carbohydrates with the enzyme under conditions which allow oxidation. Those conditions are known to the skilled person and can be optimized by routine tests. Moreover, as is also known to the skilled person, enzyme activity can be optimized by adapting the conditions. This can be done in a known manner. The oxidized products can be isolated as is known in the art.

A further object of the present invention is a preparation for the selective oxidation of terminal hydroxy or aldehyde groups in a carbohydrate of formula (I) as defined in claim 1, which preparation comprises NAD⁺ dependent short chain dehydrogenase/reductase from *Sphingomonas species A1* and/or a homolog thereof optionally an NAD⁺/NADH modulating a compound.

In one embodiment the co-factor regulating compound is an NADH abstracting compound.

Furthermore another subject of the present invention is the use of NAD⁺-dependent short chain dehydrogenase/reductase from *Sphingomonas species A1* or a homolog thereof for the preparation of L-sugar, in particular L-gulose, or aldaric acid, in particular glucaric or galactaric acid.

A further embodiment of the present invention is a process for producing an aldose or an acid of the following formula (II):

**R⁴-[CR¹R²]ₙ-CHOH-R³**

from a substrate of the formula (III):

**R⁶-[CR¹R²]ₙ-CHOH-R⁵**

wherein the process comprises an enzymatically catalysed oxidation of the terminal C-atom in R⁵ in the substrate of formula III, and wherein the enzyme is the NAD⁺-dependent short chain dehydrogenase/reductase, preferably the short chain dehydrogenase/reductase from *Sphingomonas species A1* and wherein
R¹ and R² is independently hydrogen or hydroxyl
R³ is -COOH or-CHO,
R⁴ is -CH₂OH or-COOH,
R⁵ is -CH₂OH or-CHO,
R⁶ is-CH₂OH or -COOH, and
n is a whole number from 0 to 6.

The "terminal C-atom" in the sense of the formula III is the C-atom of R⁵ and can be an aldehyde group -CHO or an alkoxygroup -CH₂OH.

In a further embodiment the C atom adjacent to R⁵ in the substrate of formula III is a chiral center and the chiral center has the stereochemical configuration D (D-CHOH).

A further embodiment of the invention is the a process for producing an aldose or an acid of the following formula (II) from a substrate of the formula (III) wherein the n in formula II or III is a whole number from 0 to 4 or from 0 to 2.

In a preferred embodiment R³ is -CHO and R⁴, R⁵ and R⁶ are -CH₂OH, more preferred the aldose of formula II is L-gulose and the substrate of the formula III is D-sorbitol or wherein the aldose of formula II is L-gyceraldehyde and the substrate of the formula III is glycerol or wherein the aldose of formula II is mannose and the substrate of the formula III is mannitol.

Another embodiment of the invention is a process for producing an uronic acid of formula II from an aldonic acid of formula III, wherein R³ is -CHO, R⁴ is -COOH, R⁵ is - CH₂OH and R⁶ is -COOH. More preferred the uronic acid is L-guluronate, D-mannuronate, L-alluronate, L-galacturonate, D-lyxuronate and L-xyluronate and the corresponding substrate of formula III is an aldonic acid and is in the same order D-gluconate, D-mannonate, D-allonate, D-galactonate, D-arabonate or D-xylonate.

Subject of the invention is also a process for producing an aldaric acid of formula II from an aldonic acid of formula III, wherein R³ and R⁴ is-COOH, R⁵ is -CH₂OH and R⁶ is - COOH. More preferred the aldaric acid of formula II is the glucaric, mannaric, allonaric, altronaric, arabic, xylaric or an talaric acid and the corresponding substrate of formula III is an aldonic acid and is in the same order D-gluconate, D-mannonate, D-allonate or D-altronate, D-arabonate or D-xylonate or D-talonate.

An further subject of the invention is a process for producing an aldaric acid of formula II from a uronic acid of formula III, wherein R³ and R⁴ is -COOH, R⁵ is -CHO and R⁶ is - COOH. More preferred the acid of formula II is an aldaric acid and is preferably glucaric or galactaric acid and the corresponding substrate of formula III is in the same order D-glucuronate or D-galacturonate.

Another aspect of the present invention is a preparation for use in one of the preceding processes characterised in that the preparation comprises an NAD⁺-dependent short chain dehydrogenase / reductase enzyme isolated from *Spingomonas species A1* or a homolog thereof and optionally an equilibrium shifting compound, preferably a NAD⁺/NADH cofactor modulating compound, more preferably a NADH abstracting compound.

A further embodiment of the invention is the use of an NAD⁺-dependent short chain dehydrogenase/reductase enzyme from *Sphingomonas species A1* or or a homolog thereof, ora preparation comprising an NAD⁺-dependent short chain dehydrogenase/reductase enzyme from *Sphingomonas species A1* in any one of the above described processes.

The present invention is further explained in the following examples, which however are not to be interpreted as limiting.

### Examples

### Reagents

Restriction enzymes, alkaline phosphatase, T4 ligase and Taq polymerase were obtained from New England Biolabs. Oligonucleotides were from biomers.net GmbH. All chemicals were of analytical grade or higher quality and purchased from Carbosynth, Sigma-Aldrich, Alfa Aesar, Serva or Carl Roth. For protein purification equipment (including columns) from GE Healthcare was used.

### Example 1: Preparation of aldonic acids and dehydrated aldonic acids

Aldonic acids were prepared from their corresponding sugars by oxidation using a gold catalyst (J213 XI/D 0.5 %, 0.5 % Au on Al₂O₃ powder, kindly provided from Evonik Industries, Germany). The procedure has been described for L-arabinose, D-xylose, D-galactose and D-glucose (Sperl 2016) as well as D-ribose, D-lyxose, D-mannose, L-rhamnose, N-acetyl-glucosamine (Mirescu 2007) and could be adapted for the oxidation of D-allose, D-altrose, L-gulose, D-talose, D-arabinose, D-erythrose, D-threose, 2-deoxy-D-glucose, and 3-deoxy-D-glucose. The procedure was a as follows: Reactions were carried out in a temperature controlled glass reactor (total volume 50 ml, initial reaction volume 30 ml) equipped with a reflux condenser, a pH electrode and a burette for base dosage, a 21 G needle as oxygen inlet and a magnetic stirrer. Prior to the start of the reaction the sugar solution was stirred at the desired pH value inside the reactor. This solution was heated to 50 °C and oxygen was supplied at a flow rate of 40 ml min⁻¹. The reactions were then started by adding an appropriate amount of gold catalyst. During the reaction, the oxygen supply was maintained at the same flow rate and the pH value was kept constant with an automatic titrator (TitroLine 7000, SI Analytics, Germany) by adding aqueous NaOH. During the course of the reaction, the amount of added NaOH was monitored. Conversion and selectivity were checked with an HPLC system as described below.

For kinetic measurements, the aldonic acids were purified (preparative scale 200-1080 mg). For this the resulting solution was loaded to a Dowex (1x8, 200-400 mesh) anion-exchange column, which was equilibrated with ammonium bicarbonate. The column was washed with three column volumes H₂O and the sugar acids were eluted with 100 mM ammonium bicarbonate (pH 10.4). The pooled fractions containing sugar acids were lyophilized and dissolved in H₂O to give a concentration of 10 mg/mL. This solution was loaded to a Dowex (50Wx8-400, 200-400 mesh) cation-exchange column in the Na⁺-form. Elution with H₂O and lyophilization gave the sugar acids as sodium salt. Karl Fischer titrations were performed to analyze the water content and to calculate standard concentrations accordingly.

The keto-deoxy sugars KDG, 2-keto-3-deoxy-L-pentonate and 2-keto-3-deoxy-D-pentonate were obtained from D-gluconate, L-arabinonate and D-xylonate, respectively using the enzyme dihydroxyacid dehydratase from *Sulfolobus solfataricus.* The detailed procedure is described by Sperl et al. (2016).

The following aldonic acids were synthesized by oxidation of the corresponding sugars using a gold catalyst: D-erythronate, D-threonate, D-arabonate, D-lyxonate, D-ribonate, D-xylonate, D-allonate, D-altronate, D-mannonate, L-gulonate, D-galactonate, D-talonate, 2-deoxy-D-gluconate, and 3-deoxy-D-gluconate. The optimal ratios of sugar to catalyst
(Table 1) were found by stepwise addition of the catalyst to the reaction mixture with online monitoring of the reaction using a titrator.

**Table 1: Synthesis of aldonic acids**

| **Sugar** | **Au-catalyst (mg)/ sugar (mmol)** | **conversion (%)** | **water content of product (%)** | **comment** |
|---|---|---|---|---|
| **D-erythrose** | 180 | 92.5 | 11.38 | |
| **D-threose** | 60 | 99.8 | 17.54 | |
| **D-arabinose** | 10 | 99.7 | 2.22 | |
| **D-lyxose** | 27 | 98.3 | 12.16 | |
| **D-ribose** | 30 | 98.8 | 11.93 | |
| **D-xylose** | 20 | 100 | 9.41 | |
| **D-allose** | 60 | 99.3 | 15.37 | |
| **D-altrose** | 40 | 97.1 | 12.60 | |
| **D-mannose** | 30 | 92.0 | 1.94 | |
| **D-galactose** | 8 | 100 | 11.94 | |
| **D-talose** | 60.5 | 93.5 | 12.55 | |
| **2-deoxy-D-glucose** | 54.2 | 96.7 | | Not optimized |
| **3-deoxy-D-glucose** | 75 | 95 | | Not optimized |

### Example 2: Construction of plasmid for overexpression of SpsADH

The DNA sequence encoding for alcohol dehydrogenase from *Sphingomonas species* A1 (SpsADH, Genebank™ accession number BAP40335.9) was synthesized as a GeneArt®Strings DNA-fragment with optimized codon usage for expression in *E. coli* and restrictions sites for *Nde*I and *Xho*I (Life Technologies). The fragment was first cloned into pJET1.2 by blunt end ligation using the kit from Thermo Fisher Scientific. Then the gene was cloned into pET28a(+) (Novagen) with an N-terminal His₆-tag yielding the plasmid pET28a-NHis-*spsadh*. Multiplication of the plasmids was performed in *E. coli* DH5α (Stratagene) in LB medium containing 30 µg/mL kanamycin.

### Example 3: Expression and purification of SpsADH

For small amounts, expression of *spsADH* was performed in *E. coli* BL21(DE3) (Novagen) containing the plasmid of interest in 1 L autoinduction medium containing 100 µg/mL kanamycin (Studier, 2005). The preculture was incubated in 20 mL LB medium with 30 µg/mL kanamycin at 37 °C overnight on a rotary shaker (180 rpm). Expression cultures were inoculated with the overnight culture at an OD₆₀₀ of 0.1. Incubation was performed for 3 h at 37 °C followed by incubation for 21 h at 16 °C. Cells were harvested by centrifugation and stored at -20 °C. For large amounts, expression of *spsadh* was performed using a 2 L Biostat Cplus bioreactor (Sartorius Stedim, Germany) in a batch cultivation process. The fermentation parameters were as follows: 2 L autoinduction medium containing 100 µg/mL kanamycin were inoculated with 10 % of a preculture grown overnight. The temperature was set at 37 °C until an OD₆₀₀ of 9 was reached, then it was decreased to 16 °C for 24 h. The pH was held constant at 7 and pO2 at 30 % with variable stirring speed and aeration. Cells were harvested by centrifugation and stored at -20 °C.

For purification of SpsADH, cells were resuspended in 50 mM sodium phosphate buffer (pH 8.0, 20 mM imidazole, 500 mM NaCl, and 10 % glycerol). Crude extracts were prepared on ice by ultrasonication (Hielscher Ultrasonics, sonotrode LS24d10): 3 cycles of 15 min pulsing (0.6 ms, 0.4 ms pause) at 80 % amplitude. The insoluble fraction of the lysate was removed by centrifugation (20,000 rpm for 40 min at 4 °C) and the supernatant was then applied to an IMAC affinity resin column (5 ml HisTrap™ FF) equilibrated with the resuspension buffer using the ÄKTA Purifier-system. The column was washed with 5 column volumes (CV) of resuspension buffer and the His-tagged protein was eluted in a gradient of 10 CV from 0 to 100 % elution buffer (50 mM sodium phosphate buffer pH 8.0, 500 mM imidazole, 500 mM NaCl, and 10 % glycerol). Aliquots of the eluted fractions were subjected to 12 % SDS-PAGE described by Laemmli (1970). The molecular weight was calculated using the ProtParam tool (Gasteiger 2005): 29,500 Da, including the additional amino acids of the N-terminal His₆-tag. The fractions containing the eluted protein were pooled and the protein was desalted using a HiPrep™ 26/10 Desalting column, which was preliminary equilibrated with 50 mM ammonium bicarbonate (AbC) pH 7.9. The protein concentration was determined by UV-spectroscopy (Nanophotometer, Implen) at 280 nm using the extinction coefficient (ε₂₈₀) calculated with the ProtParam tool (assuming all cysteins are reduced): 18,450 M⁻¹cm⁻¹. Aliquots of the protein solution were shock-frozen in liquid nitrogen and stored at -80 °C.

### Example 4: Enzyme assays for determining the activity of SpsADH

The activity of SpsADH was determined photometrically by monitoring the increase/decrease of NAD⁺/NADH at 340 nm using the Infinite 200 pro photometer (Tecan Group Ltd.). Reactions were performed at 25 C in triplicate using 96-well microtiter plates. For determination of the substrate scope, the reaction mixtures contained 50 mM ammonium bicarbonate, pH 7.9, 0.3 mM NADH (or 1 mM NAD⁺), a defined alcohol, aldehyde, aldonic acid or uronic acid as substrate and the purified enzyme. One unit of enzyme activity was defined as the amount of protein that reduces (oxidizes) 1 µmol of NAD(H)/min at 37 °C. Calculation of Michaelis-Menten kinetics for determination of Kₘ and vₘₐₓ was done with Sigma-Plot 11.0 (Systat Software).

For product identification, enzyme assays were performed over night with the addition of the cofactor recycling enzyme NADH oxidase (NOX) from *Lactobacillus pentosus.* Expression, purification and activation with FAD was performed as described previously (Nowak et al., 2015). Reaction mixtures contained 50 mM AbC pH 7.9, 25 mM substrate, 5 mM NAD⁺ and purified SpsADH. NOX was added three times due to stability issues (Beer et al., 2017).

### Example 5: HPLC analysis of carbohydrates

Aldonic and aldaric acids were separated by HPLC, using an Ultimate-3000 HPLC system (Dionex, Idstein, Germany), equipped with autosampler (WPS 3000TRS), a column compartment (TCC3000RS), and a diode array detector (DAD 3000RS). The column Metrosep A Supp10-250/40 column (250 mm, particle size 4.6 mm; Metrohm, Filderstadt, Germany) at 65 °C was used for separation by isocratic elution with 30 mM ammonium bicarbonate (pH 10.4) as mobile phase at 0.2 mL min⁻¹. Samples were diluted in water, filtered (10 kDa MWCO, modified PES; VWR, Darmstadt, Germany) and 10 µL were applied on the column. Data was analyzed with Dionex Chromeleon software.

Uronic acids were analyzed by HPLC/MS as PMP (1-phenyl-3-methyl-5-pyrazolone) derivatives using the method of Rühmann et al. (2014).

Samples were diluted in water and derivatized with 0.1 M PMP and 0.4% ammonium hydroxide in methanol. After 100 min at 70 °C the reaction was stopped with 16.7mM acetic acid and filtered (Restek, 0,22 µm, PVDF). When elution of the uronic acid was expected before three min, the sample was extracted with chloroform (three times) to reduce excess PMP and then filtered.

The HPLC system (Ultimate 3000RS, Dionex) was composed of a degasser (SRD 3400), a pump module (HPG 3400RS), an autosampler (WPS 3000TRS), a column compartment (TCC3000RS), a diode array detector (DAD 3000RS) and an ESI-iontrap unit (HCT, Bruker). Data was collected and analyzed with BrukerHystar software. The column (Gravity C18, 100 mm length, 2 mm i.d.; 1.8 µm particle size; Macherey-Nagel) was operated at 50 °C with a mobile phase A (5 mM ammonium acetate buffer, pH 5.6, with 15 % acetonitrile) and a chromatographic flow rate of 0.6 mL/min. The gradient (mobile phase B containing pure acetonitrile) was as follows: start of mobile phase B at 1%, with increase to 5 % over 5 min, hold for 2 min, then increase to 18 % over 1 min. The gradient was further increased to 40 % over 0.3 min, held for 2 min and returned within 0.2 min to starting conditions for 1.5 min. If samples were not extracted, the first 3 min of chromatographic flow were refused by a switch valve behind the UV-detector (245 nm). Before entering ESI-MS the flow was splitted 1:20 (Accurate-Post-Column-Splitter, Dionex). Temperature of the autosampler was set to 20 °C and an injection volume of 10 µL was used.

ESI-ion-trap parameters: The ion-trap operated in the ultra scan mode (26,000 m/z/s) from 50 to 1000 m/z. The ICC target was set to 200,000 with a maximum accumulation time of 50 ms and four averages. The ion source parameters were set as follows: capillary voltage 4 kV, dry temperature 325 °C, nebulizer pressure 40 psi and dry gas flow 6 L/min. Auto-MS-mode with the smart target mass of 600 m/z and a MS/MS fragmentation amplitude of 0.5 V was used. The quantification was performed by using the extracted ion chromatograms (EIC) of the m/z value corresponding to the protonated molecules.

### Example 6: Oxidation of C6 of aldonic acids

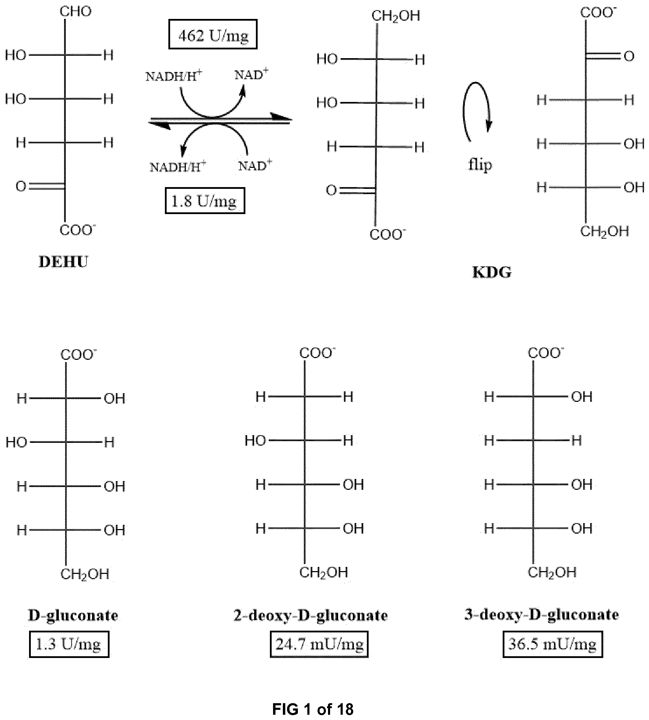

SpsADH has been described for the reduction of DEHU to KDG, by reducing the terminal aldehyde (C6 in regard of KDG. However oxidation reactions have not been described. Inventors analyzed SpsADH with its native substrate KDG and found very low oxidative activity. Surprisingly when the kinetic parameters for the analogue gluconate, which has hydroxyl-groups at C2 und C3 was measured oxidative activity was identified: vₘₐₓ = 1.3 ± 0.0 U/mg; Kₘ = 15.7 ± 1.6 mM. To substantiate these results, 2-deoxygluconate and 3-deoxygluconate were synthesized and tested. These substrates are lacking a hydroxyl-group at C2 or C3 compared to gluconate, respectively. Surprisingly, only very low activities of 24.7 ± 0.6 and 36.5 ± 0.2 mU/mg, respectively were detected at 25 mM substrate concentration (Figure 1).

**Table 2: Kinetic parameters for oxidation of KDG by SpsADH**

| | **Kₘ [mM]** | **vₘₐₓ [U/mg]** | **k_{cat} [s⁻¹]** | **k_{cat}/Kₘ (M⁻¹ s⁻¹)** | **k_{cat}/Kₘ (%)** |
|---|---|---|---|---|---|
| **KDG** | 20.9 ± 3.16 | 1.81 ± 0.009 | 0.89 ± 0.047 | 42.5 ± 8.68 | |

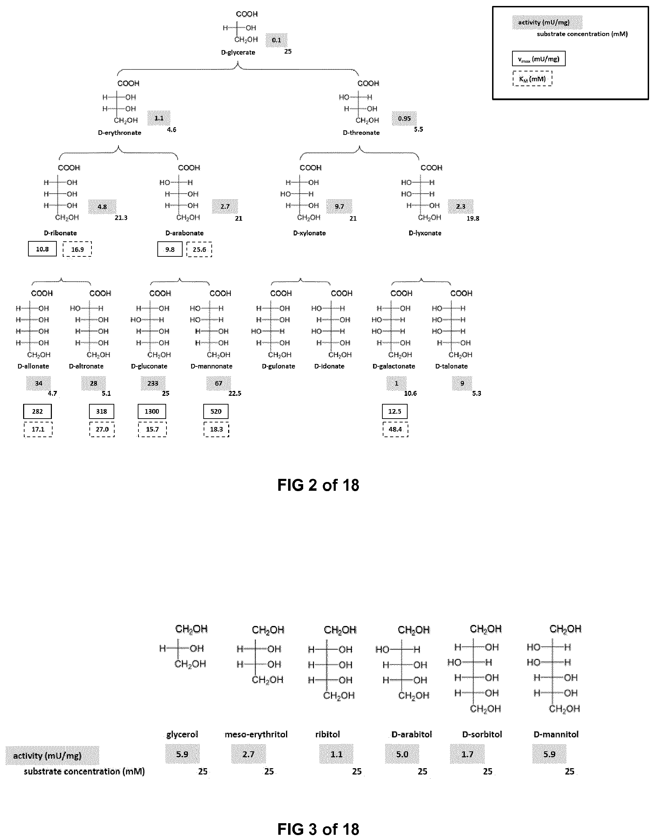

To investigate the substrate specificity C6- and C5-aldonic acids were synthesized and tested as substrates for SpsADH (Figure 2). C6-aldonates D-allonate, D-altronate, D-gluconate, and D-mannonate, D-galactonate, and D-talonate were oxidized by spsADH to uronic acids (and showed enzyme activities from 28 to 233 mU/mg at 1 to 25 mM substrate concentration. The conversion of aldonates to uronic acids was exemplified also with C5-aldonates. in this group D-xylonate, D-ribonate, D-arabinonate and D-lyxonate were used as substrates and could be converted to uronic acids. The high reactive erythro-group aldonates (C5 and C6) were analyzed in regard of Kₘ and vₘₐₓ. The lowest Kₘ as well as the highest vₘₐₓ were measured with D-gluconate (15.7 mM). However, the affinity was comparable with those of D-allonate, D-mannonate, and D-ribonate (17.1, 18.3, and 16.9 mM, respectively). D-Arabinonate and D-altronate showed affinities of 25.6 and 27 mM, respectively. The catalytic efficiency for the various substrates is shown in Table 3. C₄ and C₃ aldonates (D-erythronate, D-threonate, D-glycerate) could also be oxidized by SpsADH to uronic acids.

**Table 3: Kinetic parameters for oxidation of aldaric acids by SpsADH**

| | **KM [mM]** | **vmax [U/mg]** | **kcat [s-1]** | **kcat/KM (M-1 s-1)** | **kcat/KM (%)** |
|---|---|---|---|---|---|
| D-gluconate | 15.7 ± 1.6 | 1.28 ± 0.04 | 0.63 ± 0.02 | 40.0 ± 5.4 | 100.0 |
| D-mannonate | 18.3 ± 1.3 | 0.52 ± 0.01 | 0.26 ± 0.00 | 13.9 ± 1.2 | 34.8 |
| D-allonate | 17.1 ± 2.4 | 0.28 ± 0.01 | 0.14 ± 0.01 | 8.1 ± 1.4 | 20.2 |
| D-altronate | 27.0 ± 4.2 | 0.32 ± 0.01 | 0.16 ± 0.01 | 5.8 ± 1.2 | 14.5 |
| D-ribonate | 16.9 ± 1.4 | 0.01 ± 0.00 | 0.01 ± 0.00 | 0.3 ± 0.1 | 0.8 |
| D-arabinonate | 25.6 ± 2.6 | 0.01 ± 0.00 | 0.01 ± 0.00 | 0.2 ± 0.0 | 0.5 |

### Example 7: Oxidation of C6 of aldoses and reduction of C1 of aldoses

The next group of substrates tested were C6 sugars. Here, C1 carries an aldehyde group instead of a carboxylic acid. However, no oxidative activity was detected for any of the C6 sugars. C6 sugars build hemiacetals in which the free -OH group on the C-atom adjacent to the end terminal C-atom is part of the cyclic moiety. It is estimated that this -OH group is important for the oxidative reactivity of SpsADH. To verify this hypothesis these substrates were tested for reduction of the aldehyde group, which also is not available in the hemiacetal form and therefore these sugars should not be susceptible to reduction. The reductive activity with C6 sugars indeed was hardly measurable. Thus, C6 sugars that are in form of a hemiacetal are not preferred substrates.

### Example 8: Oxidation at -CHO group of uronic acids

In uronic acids, only an aldehyde group at C1 is available for oxidation by SpsADH, while C6 is oxidized to the carboxylic acid. The highest oxidative activity at 25 mM substrate concentration was seen for D-glucuronate (361 mU/mg), followed by D-galacturonate (211 mU/mg), L-iduronate (8.6 mU/mg), D-mannuronate (4.0 mU/mg) and L-guluronate (0.3 mU/mg). D-glucuronate, D-galacturonate, L-iduronate, D-mannuronate have the favorable D configuration on the C atom adjacent to the terminal C-atome subject to oxidation. The L-guluronate has L configuration on this C atom and is not oxidized in significant amount (Figure 16).

### Example 9: Oxidation of terminal C atom of polyols

Polyols, where both terminal C-atoms are reduced to the hydroxyl group were tested. At 25 mM substrate concentration the D-mannitol D-sorbitol, D-arabitol, meso-erythritol and ribitol (activities from 1,1 to 5,9 mU/mg) were accepted as substrates and converted to (rare) sugars, whereas polyols with C-atom adjacent to TOG in D configuration (D-CHOH) exhibit the best activities (Figure 3).

### Example 10: Production of the rare sugar L-gulose from D-sorbitol

Production of L-gulose was performed in a volume of 50 mL in a two-necked roundbottom glass flask. One neck was used for drawing samples, the second neck for supplementing gaseous, humidified oxygen through a cannula at 20 mL/min once a day for 1 to 2 min. The reaction mixture contained 50 mM ammonium bicarbonate, 1 mM NAD⁺, 1.5 M D-sorbitol, and 0.04 U/mL of SpsADH and NOX, respectively. Kinetic parameters of NOX were determined in presence of 1.5 M D-sorbitol in 50 mM ammonium bicarbonate, and varying amounts of NADH.

The reaction took place at 25 °C (water bath) with stirring. 0.04 U/mL NOX were added multiple times over the entire duration of the experiment. Also, in order to keep the activity of SpsADH at vₘₐₓ, 3.0 g and 4.5 g D-sorbitol were added after 117 h and 200 h, respectively.

Samples for HPLC analysis were ultrafiltrated with spin filters (10 kDa MWCO, modified PES; VWR) to remove enzymes and stop the reaction.

The experiment was stopped after L-gulose concentrations did not rise any more. Due to an impurity of the used D-sorbitol charge, glucose had to be removed from the reaction mixture. Therefore, the reaction mixture was incubated with glucose oxidase and catalase to convert glucose to gluconate, which was then removed by anion exchange chromatography using DOWEX 1x8, 200-400 mesh. Pure L-gulose was then obtained by cation-exchange chromatography (Dowex 50Wx8-400, 200-400 mesh in the Na⁺-form). Elution monitoring and quantification was performed using an HPLC system (Dionex®, Sunnyvale, CA, USA) equipped with a Rezex ROA-H⁺ column (Phenomenex®, Torrance, CA, USA), and a refractive index detector (RI 101, Shodex, Tokyo, Japan). The mobile phase (sulfuric acid, 2.5 mM) was set to a flow rate of 0.5 mL min⁻¹ at an oven temperature of 70 °C. Quantitative calculations were referred to an external standard. Purification of L-gulose was possible with a recovery of 66 % pure L-gulose (Figures 17).

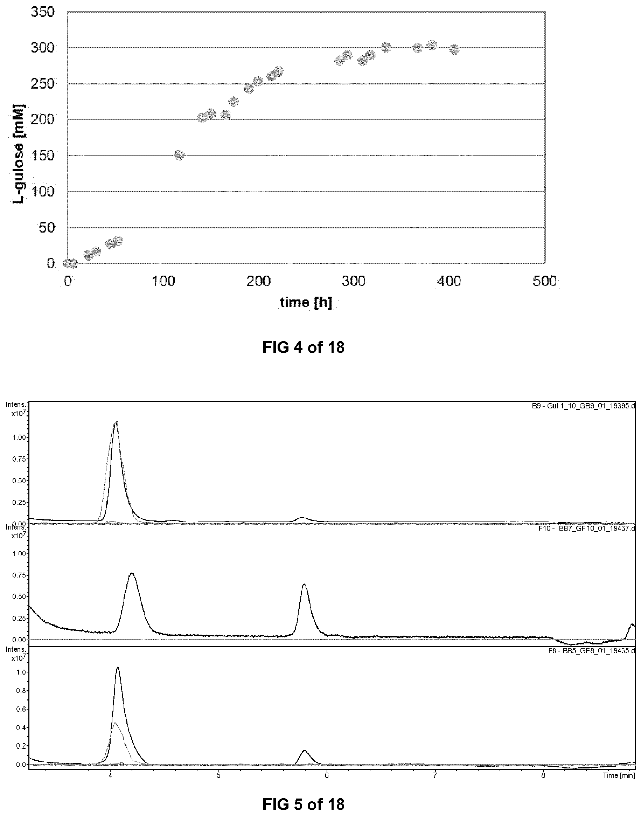

SpsADH could be used for the synthesis of the rare and high-priced sugar L-gulose from available and cheap D-sorbitol. From kinetic analysis, it was found an extremely high K_{M} of 350± 19 mM and fairly low vₘₐₓ of 6.2 ± 0.1 mU/mg. However, within 334 h 300 mM L-gulose were produced, resulting in a space-time-yield (STY) of 0.9 mM h⁻¹ or 3.9 g L⁻¹ d⁻¹ (Figure 4).

### Example 11: Stability and total turnover

As SpsADH exhibits only low promiscuous activities, the stability of the enzyme is very important to ensure complete conversion by extended incubation. Therefore, we analyzed the enzyme at 25 °C and found the half-life to be 20 h. In case of oxidation of D-gluconate to L-guluronic acid, this gives a total turnover number of 28.300.

### Example 12: Product analyses

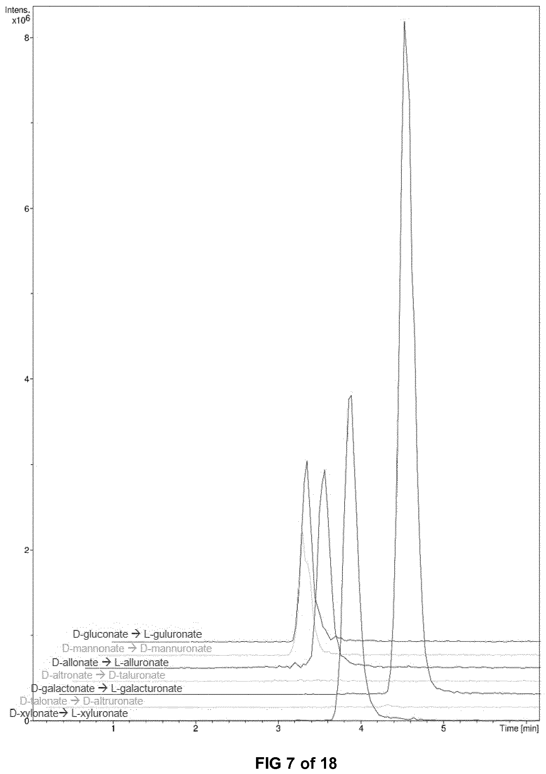

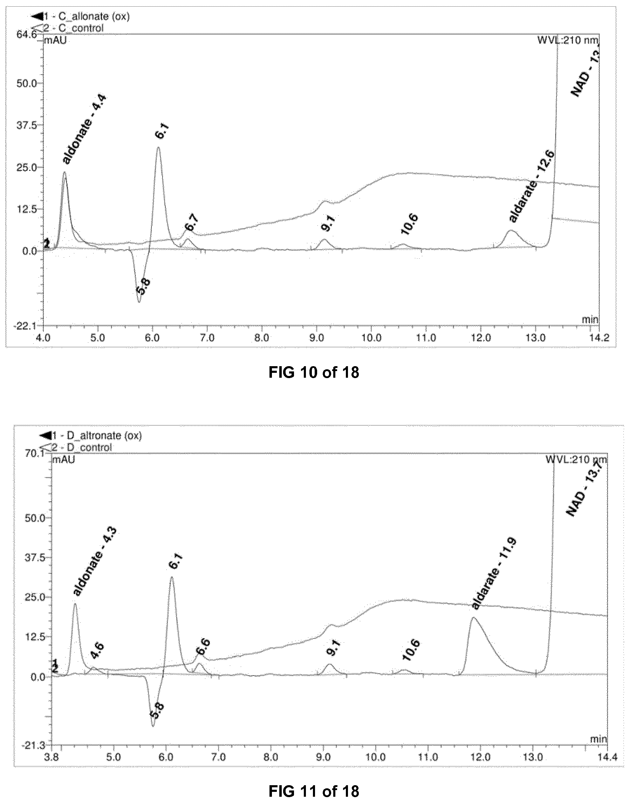

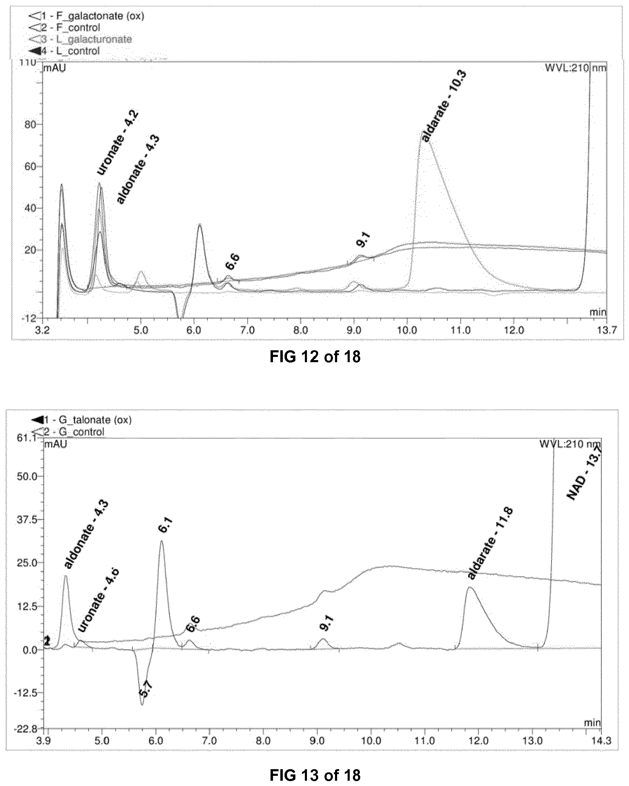

The product formation from oxidation of polyols and aldonic acids was verified by HPLC analysis (see Example 4). The formation of uronic acids (Figure 7): L-guluronate (from D-gluconate), D-mannuronate (from D-mannonate), L-alluronate (from D-allonate), L-galacturonate (from D-galactonate), and D-lyxuronate (from D-arabonate), and L-xyluronate (from D-xylonate) was proved. The uronic acids D-taluronate and D-altruronate were converted to the corresponding aldaric acids (Figures 11 and 13). For all tested aldonates the formation of the corresponding aldaric acids was shown.

As NADH oxidase was also present in the reactions for product analyses, it oxidized the cofactor in favor of substrate oxidation, in case of the oxidation of the aldaric acid D-gluconate, the uronic acid L-guluronate was formed and oxidized further to the aldaric acid glucarate. This resulted from the NADH oxidase present in the reaction. NADH oxidase oxidized the cofactor in favor of substrate oxidation, resulting in the oxidation of L-guluronate to glucarate. With this reaction being essentially irreversible, the equilibrium of the reaction was shifted toward glucarate.

In case of the sorbitol oxidation, we could prove that SpsADH specifically oxidizes the C6 carbon with adjacent C-atom in D configuration, yielding L-gulose as the sole product (oxidation at C1 would give D-glucose) (Figure 5).

### REFERENCES

Arora, B., Mukherjee, J., Gupta, M. N., 2014. Enzyme promiscuity: using the dark side of enzyme specificity in white Biotechnology. Sustainable Chemical Processes. 2.
Beer, B., Pick, A., Sieber, V., 2017. In vitro metabolic engineering for the production of α-ketoglutarate. Metab. Eng. 40, 5-13.
Benach, J., Filling, C., Oppermann, U. C., Roversi, P., Bricogne, G., Berndt, K. D., Jornvall, H., Ladenstein, R., 2002. Structure of bacterial 3beta/17beta-hydroxysteroid dehydrogenase at 1.2 A resolution: a model for multiple steroid recognition. Biochemistry. 41, 14659-68.
Granström, T. B., Takata, G., Tokuda, M., Izumori, K., 2004. Izumoring: A novel and complete strategy for bioproduction of rare sugars. J. Biosci. Bioeng. 97, 89-94.
Hashimoto, W., Kawai, S., Murata, K., 2010. Bacterial supersystem for alginate import/metabolism and its environmental and bioenergy applications. Bioengineered Bugs. 1, 97-109.
Hirano, J., Miyamoto, K., Ohta, H., 2005. Purification and characterization of the alcohol dehydrogenase with a broad substrate specificity originated from 2-phenylethanol-assimilating Brevibacterium sp. KU 1309. J. Biosci. Bioeng. 100, 318-22.
Inoue, A., Nishiyama, R., Mochizuki, S., Ojima, T., 2015. Identification of a 4-deoxy-L-erythro-5-hexoseulose uronic acid reductase, FIRed, in an alginolytic bacterium Flavobacterium sp. strain UMI-01. Mar. Drugs. 13, 493-508, 16 pp.
Izumori, K., 2006. Izumoring: a strategy for bioproduction of all hexoses. J. Biotechnol. 124, 717-22.
Kavanagh, K. L., Jornvall, H., Persson, B., Oppermann, U., 2008. Medium- and short-chain dehydrogenase/reductase gene and protein families : the SDR superfamily: functional and structural diversity within a family of metabolic and regulatory enzymes. Cell. Mol. Life Sci. 65, 3895-906.
Kazlauskas, R. J., 2005. Enhancing catalytic promiscuity for biocatalysis. Curr. Opin. Chem. Biol. 9, 195-201.
Khersonsky, O., Tawfik, D. S., 2010. Enzyme promiscuity: mechanistic and evolutionary perspective. Annu. Rev. Biochem. 79.
Kim, K. H., Ha, B. H., Kim, S. J., Hong, S. K., Hwang, K. Y., Kim, E. E., 2011. Crystal structures of Enoyl-ACP reductases I (FabI) and III (FabL) from B. subtilis. J. Mol. Biol. 406, 403-15.
Koropatkin, N. M., Holden, H. M., 2005. Structure of CDP-D-glucose 4,6-dehydratase from Salmonella typhi complexed with CDP-D-xylose. Acta Crystallogr D Biol Crystallogr. 61, 365-73.
Laemmli, U. K., 1970. Cleavage of Structural Proteins during the Assembly of the Head of Bacteriophage T4. Nature. 227, 680-685.
Lee, H. H., Moon, J., Suh, S. W., 2007. Crystal structure of the Helicobacter pylori enoyl-acyl carrier protein reductase in complex with hydroxydiphenyl ether compounds, triclosan and diclosan. Proteins. 69, 691-4.
Li, Z., Wu, X., Cai, L., Duan, S., Liu, J., Yuan, P., Nakanishi, H., Gao, X. D., 2015. Enzymatic synthesis of rare sugars with L-rhamnulose-1-phosphate aldolase from Thermotoga maritima MSB8. Bioorg. Med. Chem. Lett. 25, 3980-3.
Mochizuki, S., Nishiyama, R., Inoue, A., Ojima, T., 2015. A Novel Aldo-Keto Reductase, HdRed, from the Pacific Abalone Haliotis discus hannai, Which Reduces Alginate-derived 4-Deoxy-L-erythro-5-hexoseulose Uronic Acid to 2-Keto-3-deoxy-D-gluconate. J. Biol. Chem. 290, 30962-74.
Nowak, C., Beer, B. C., Pick, A., Roth, T., Lommes, P., Sieber, V., 2015. A water-forming NADH oxidase from Lactobacillus pentosus and its potential application in the regeneration of synthetic biomimetic cofactors. Front. Microbiol. 6, 957.
O'Brien, P. J., Herschlag, D., 1999. Catalytic promiscuity and the evolution of new enzymatic activities. Chem. Biol. 6, R91-R105.
Pennacchio, A., Giordano, A., Pucci, B., Rossi, M., Raia, C. A., 2010. Biochemical characterization of a recombinant short-chain NAD(H)-dependent dehydrogenase/reductase from Sulfolobus acidocaldarius. Extremophiles. 14, 193-204.
Preiss, J., Ashwell, G., 1962. Alginic acid metabolism in bacteria. II. The enzymatic reduction of 4-deoxy-L-erythro-5-hexoseulose uronic acid to 2-keto-3-deoxy-D-gluconic acid. J. Biol. Chem. 237, 317-21.
Price, A. C., Zhang, Y. M., Rock, C. O., White, S. W., 2004. Cofactor-induced conformational rearrangements establish a catalytically competent active site and a proton relay conduit in FabG. Structure. 12,417-28.
Robins, K., Osorio-Lozada, A., 2012. Exploiting duality in nature: industrial examples of enzymatic oxidation and reduction reactions. Catalysis Science & Technology. 2, 1524-1530.
Ruhmann, B., Schmid, J., Sieber, V., 2014. Fast carbohydrate analysis via liquid chromatography coupled with ultra violet and electrospray ionization ion trap detection in 96-well format. J Chromatogr A.
Sperl, J. M., Carsten, J. M., Guterl, J.-K., Lommes, P., Sieber, V., 2016. Reaction Design for the Compartmented Combination of Heterogeneous and Enzyme Catalysis. ACS Catal. 6, 6329-6334.
Stekhanova, T. N., Mardanov, A. V., Bezsudnova, E. Y., Gumerov, V. M., Ravin, N. V., Skryabin, K. G., Popov, V. O., 2010. Characterization of a thermostable short-chain alcohol dehydrogenase from the hyperthermophilic archaeon Thermococcus sibiricus. Appl. Environ. Microbiol. 76, 4096-8.
Studier, F. W., 2005. Protein production by auto-induction in high-density shaking cultures. Protein Expr. Purif. 41, 207-234.
Svegelj, M. B., Stojan, J., Rizner, T. L., 2012. The role of Ala231 and Trp227 in the substrate specificities of fungal 17beta-hydroxysteroid dehydrogenase and trihydroxynaphthalene reductase: Steroids versus smaller substrates. J. Steroid Biochem. Mol. Biol. 129, 92-8.
Takase, R., Mikami, B., Kawai, S., Murata, K., Hashimoto, W., 2014. Structure-based conversion of the coenzyme requirement of a short-chain dehydrogenase/reductase involved in bacterial alginate metabolism. J. Biol. Chem.
Takase, R., Ochiai, A., Mikami, B., Hashimoto, W., Murata, K., 2010. Molecular identification of unsaturated uronate reductase prerequisite for alginate metabolism in Sphingomonas sp. A1. Biochim. Biophys. Acta. 1804, 1925-1936.
Turner, N. J., 2009. Directed evolution drives the next generation of biocatalysts. Nat. Chem. Biol. 5, 567-573.
Wargacki, A. J., Leonard, E., Win, M. N., Regitsky, D. D., Santos, C. N., Kim, P. B., Cooper, S. R., Raisner, R. M., Herman, A., Sivitz, A. B., Lakshmanaswamy, A., Kashiyama, Y., Baker, D., Yoshikuni, Y., 2012. An engineered microbial platform for direct biofuel production from brown macroalgae. Science. 335, 308-13.
Woodyer, R. D., Christ, T. N., Deweese, K. A., 2010. Single-step bioconversion for the preparation ofL-gulose and L-galactose. Carbohydr Res. 345, 363-8.
Yoon, R. Y., Yeom, S. J., Park, C. S., Oh, D. K., 2009. Substrate specificity of a glucose-6-phosphate isomerase from Pyrococcus furiosus for monosaccharides. Appl. Microbiol. Biotechnol. 83, 295-303.
Zaccai, N. R., Carter, L. G., Berrow, N. S., Sainsbury, S., Nettleship, J. E., Walter, T. S., Harlos, K., Owens, R. J., Wilson, K. S., Stuart, D. I., Esnouf, R. M., 2008. Crystal structure of a 3-oxoacyl-(acylcarrier protein) reductase (BA3989) from Bacillus anthracis at 2.4-A resolution. Proteins. 70, 562-7.
Zhang, Q., Peng, H., Gao, F., Liu, Y., Cheng, H., Thompson, J., Gao, G. F., 2009. Structural insight into the catalytic mechanism of gluconate 5-dehydrogenase from Streptococcus suis: Crystal structures of the substrate-free and quaternary complex enzymes. Protein Sci. 18, 294-303.

## Claims

1. A process for enzymatic oxidation of a terminal group of a carbohydrate, wherein a carbohydrate of formula (I):
**TU-X-Y-TOG**
wherein
TU is a terminal unit selected from CH₂OH, CHO, COOH, and C₁-C₄ alkyl;
X is a linear carbohydrate chain having 0 to 17 units [CR¹R²], wherein in each unit R¹ and R² independently can be hydrogen, hydroxyl, C₁-C₄ alkyl, or wherein R¹ and R² together can form a keto group,
Y is CHOH, -CHNH₂ or -CHSH and
TOG is a terminal oxidizable unit selected from -CH₂OH and -CHO;
is contacted with an NAD⁺ dependent short chain dehydrogenase/reductase for oxidation of TOG.

2. The process of claim 1 wherein the enzyme is NAD⁺ dependent short chain dehydrogenase/reductase from *Sphingomonas species A1* or a homolog thereof.

3. The process of claim 1 or 2 wherein the Y is -CHOH.

4. The process of claim 1 to 3 wherein X comprises 0 to 3 units.

5. The process of any one of the preceding claims wherein TOG is -CH₂OH and TU is - CH₂OH.

6. The process of claim 5 wherein the Y is D-CHOH.

7. The process according to claim 5 or 6 wherein the carbohydrate of formula I is D-sorbitol, glycerol or mannitol.

8. The process of any of claims 1 to 4 wherein TOG is -CH₂OH, and TU is a carboxy group.

9. The process of claim 8 wherein the Y is D-CHOH.

10. The process of claim 8 or 9 wherein the carbohydrate of formula I is an aldonic acid, in particular D-gluconate, D-mannonate, D-allonate or D-altronate, D-arabonate or D-xylonate or D-talonate.

11. The process of any of claims 1 to 4 wherein TOG is CHO, and TU is a carboxyl group.

12. The process of claim 11 wherein the carbohydrate of formula I is an uronic acid, in particular D-glucuronate,or D-galactironate.

13. A preparation for selective oxidation of terminal hydroxyl or aldehyde groups in a carbohydrate of formula I as defined in one of claims 1 to 5 comprising NAD⁺-dependent short chain dehydrogenase/reductase from *Sphingomonas species A1* and/or a homolog thereof and optionally an NAD⁺/NADH modulating compound.

14. Preparation of claim 13, wherein the modulating compound is an NADH abstracting compound.

15. Use of NAD⁺-dependent short chain dehydrogenase/reductase from *Sphingomonas species A1 or a homolog thereof* or of the preparation of claim 13 or 14 for the preparation of L-sugar, in particular L-gulose, or aldaric acid, in particular glucaric or galactaric acid.


SEQUENCE LISTING

<110> Technische Universität München

<120> Process for enzymatic oxidation of linear carbohydrates

<130> ETU170902PEP

<140> EP1719245.7
<141> 2017-10-11

<160> 1

<170> BiSSAP 1.3.6

<210> 1
<211> 798
<212> DNA
<213> Artificial Sequence

<220>
<223> codon optimized DNA sequence encoding SpsADH

<400> 1
gatctacata tgttcagcga cctgaaaggt aaacgtattc tgattaccgg tagcaccgaa 60

ggtattggta tggcaaccgc aattgaactg gcacgttatg gtgcagttgt tggtctgaat 120

agccatgttg atccggcaga tcctgcactg ctgctgggta aactgcgtga agccggtggt 180

gatggtgcat tttttcgtgc agatatcacc aaaaccgcag aatgtcagcg tctggttagc 240

gcatttgttg aacgttttga tggtattgat gtgctgatta acaatgccgg tggtctggca 300

ggtcgtagca atctggaaaa tattgatgat gccttttatg accgtgtgat ggatctgaat 360

ggtcgtagcg ttctgatgat gaccaaattt gcaattccgc atctgcgtgc aagcgcaaaa 420

gcaagcggta caaccagcgc agttattagc accggtagta ttgcagcacg tgaaggtggt 480

ggtattggtg ccggtgttta tgcagcaagc aaagcatggc tgcatgatat tcatcgtaat 540

tgggtgaaag agttcaccaa agatagcatc cgttttaaca ttgttgcacc gggtacagtt 600

gataccgcat ttcatgcaga taaaagcgac gaactgaaaa cccgtattgc aaatagcatt 660

ccgatgggtc gttttggcac cgttcaagag ctggcaccag cctatgtttt ttttgcaagc 720

catgccgcaa gcggttatat caccggtcag attctggatg ttaatggtgg tcagatttgt 780

ccgtaactcg aggatcta 798